# EUROPEAN PATENT APPLICATION

(11) **EP 0 955 032 A1**
(43) Date of publication of application: **10.11.1999**
(21) Application number: 97918361.3
(22) Date of filing: 30.04.1997
(51) Int. Cl.: A61K 7/00, A61K 7/48, A61K 7/50

(54) **COSMETIC**

(30) Priority: 02.05.1996 JP 11160396
(71) Applicant: Technoble Co., Ltd., Osaka-shi, Osaka-fu 550 (JP)
(72) Inventor: SAWAKI, Shigeru, Technoble Co., Ltd., Osaka-shi, Osaka-fu 550 (JP); YAMADA, Katsuhisa, Technoble Co., Ltd., Osaka-shi, Osaka-fu 550 (JP); NAITO, Kazufumi, Technoble Co., Ltd., Osaka-shi, Osaka-fu 550 (JP)
(74) Representative: Kinzebach, Werner, Dr.
(86) International application number: JP9701511
(87) International publication number: WO9741825

(57) **Abstract**

The cosmetic preparation inhibits the biosynthesis of melanin and onset of erythema, prevents the occurrence of inflammation, improves water content of the skin and thus, contributes to improved skin quality and to the prevention of skin aging. In addition, the preparation is less irritant and highly stable. The preparation contains the 2,5-dihydroxy benzoic acid derivatives expressed by Formula (I). 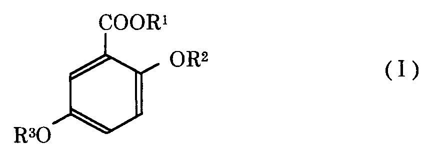 [In formula (1), R¹ is a hydrogen atom, an alkyl group with one to 20 carbons, an allylalkyl group with seven to nine carbons, an alkali metal atom or an alkaline-earth metal atom; R² and R³ are independently a hydrogen atom, a group expressed by the formula -COR⁴ (R⁴ is an alkyl group with one to five carbons, an alkenyl group with one to three carbons, a methoxy group or a ethoxy group) or a phophoryl group. However, R² and R³ should not be hydrogen atoms in an identical compound.]

## Description

### Technological field of the present invention

The present invention relates to a cosmetic preparation, specifically, a preparation that provides excellent whitening, anti-inflammatory, and moisturizing effects. The preparation not only contributes to the prevention of skin aging, but also is less irritant to the skin and highly stable. It can be preferably used in skin-care products and other products such as make-up products, toiletry products, and bathroom products.

### Description of the related arts

As the demand for cosmetic preparations with whitening, anti-inflammatory and/or moisturizing effects increases, a variety of studies have been carried out and different cosmetic preparations have been developed. However, materials such as the active ingredients used in conventional cosmetic preparations have some disadvantages: some are less safe because of irritation to the skin or are unstable. In addition, they cannot sufficiently satisfy the requirements for whitening effect, anti-inflammatory effect and moisturizing effect at the same time, and are less effective in the prevention of skin aging.

### Problems to be solved by the present invention

The present invention was developed in the light of the conventional technology described above. The object of the present invention is, therefore, to provide a cosmetic preparation that displays such excellent properties as reduction of tyrosinase activity in the pigment cell, inhibition of the biosynthesis of tyrosinase enzyme protein and subsequent inhibition of melanin formation, inhibition of erythema and chromatosis caused by ultra-violet radiation, reduction of cyclooxygenase activity inhibition of inflammation, and moisturization and enhancement of skin condition, in such a way as to serve not only to prevent skin aging, but at the same to be less irritant and endowed with greater stability.

### Means adopted to solve the problems

The present invention relates to a cosmetic preparation that contains 2,5-dihydroxy benzoic acid derivatives expressed by Formula (I): 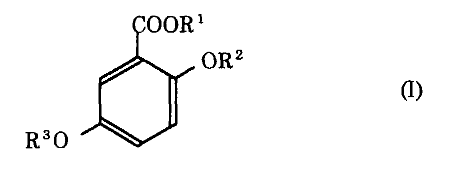 [In Formula (I), R¹ is a hydrogen atom, an alkyl group with one to 20 carbons, an allylalkyl group with seven to nine carbons, an alkali metal atom or an alkaline-earth metal atom, and R² and R³ are, independently, a hydrogen atom, a group expressed by the formula -COR⁴ (R⁴ is an alkyl group with one to five carbons, an alkenyl group with one to three carbons, a methoxy group or an ethoxy group) or a phosphoryl group (however, R² and R³ should not be hydrogen atoms in an identical compound).]

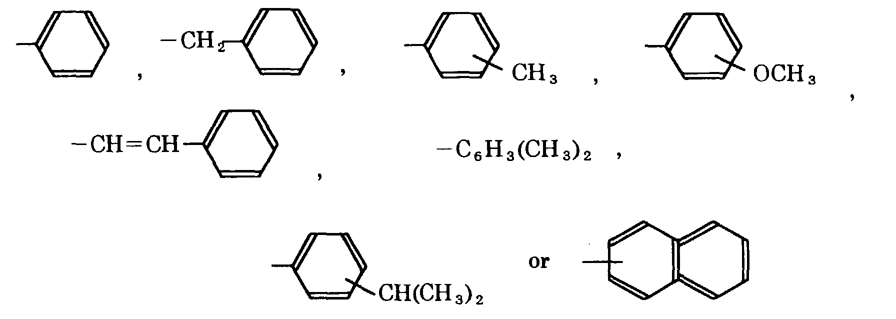

### Brief description of the drawings

Fig. 1 is a graph showing the DOPA value of each sample in Experiment 2.
Fig. 2 is a graph showing the MTT test value of each sample in Experiment 2.
Fig. 3 is a graph showing the relationship between reaction time and oxygen consumption in each sample in Experiment 3.
Fig. 4 is a graph showing the DOPA level of each sample in Experiment 5.
Fig. 5 is a graph showing the MTT level of each sample in Experiment 5.

### Detailed description of the invention

As stated above, the present invention, a cosmetic preparation, contains 2,5-dihydroxy benzoic acid derivatives as is expressed by Formula (I): 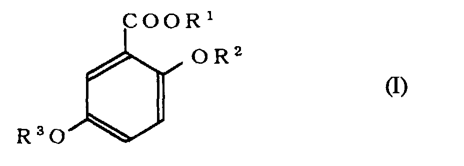 [In Formula (I), R¹ is a hydrogen atom, an alkyl group with one to 20 carbons, an allylalkyl group with seven to nine carbons, an alkali metal atom or an alkaline-earth metal atom, and R² and R³ are, independently, an hydrogen atom, a group expressed by the formula -COR⁴ (R⁴ is either an alkyl group with one to five carbons, an alkenyl group with one to three carbons, a methoxy group or an ethoxy group) or a phosphoryl group (however, R² and R³ should not be hydrogen atoms in an identical compound).]

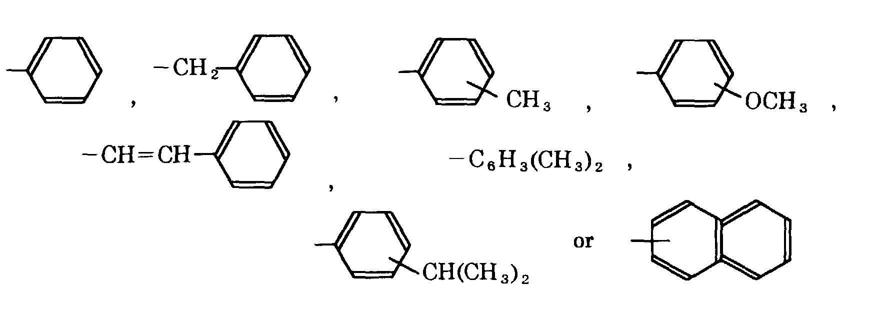

The 2,5-dihydroxy benzoic acid derivatives used in the present invention and expressed by Formula (I) are the compounds that not only add excellent whitening, anti-inflammatory ,and moisturizing effects to cosmetic preparations simultaneously, but also are less irritant to the skin and highly stable.

When R¹ is an alkyl group or an allylalkyl group in Formula (I), a group with small number of carbons is desirable for R¹, in view of the ease of synthesis and availability.

Therefore, a hydrogen atom, an alkali metal atom such as potassium and sodium, alkaline-earth metal such as magnesium, an alkyl group or a benzyl group with one to six carbons or one to three carbons respectively is desirable for R¹.

Regarding R² and R³ in Formula (I), different groups with small numbers of carbons are desirable for R² and R³ respectively, in view of the ease of synthesis and availability.

Therefore, a hydrogen atom and a group expressed by the formula -COR⁴ (refer to the aforementioned description of R⁴) is desirable for R² and R³ respectively, and an alkyl group with 1-3 carbons, an alkenyl group with 1-3 carbons, a methoxy group or ethoxy group is desirable.

The following formulas are typical examples of the 2,5-dihydroxy benzoic acid derivatives expressed by Formula (I):

The compounds expressed by Formula (II): 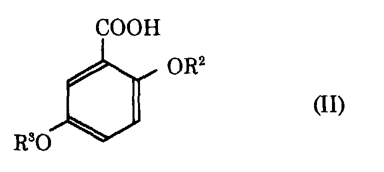 (Refer to the aforementioned description of R² and R³; However R² and R³ should not be hydrogen atoms in an identical compound.) Examples include the following:

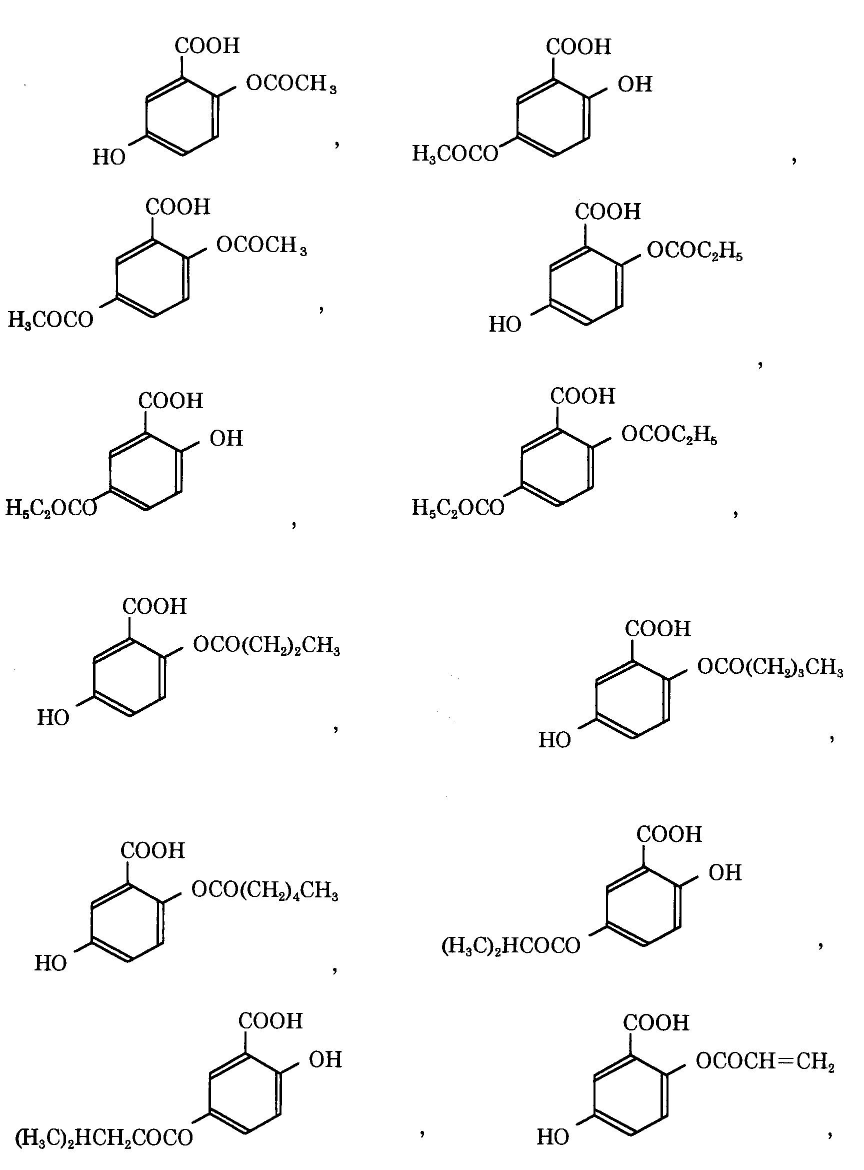

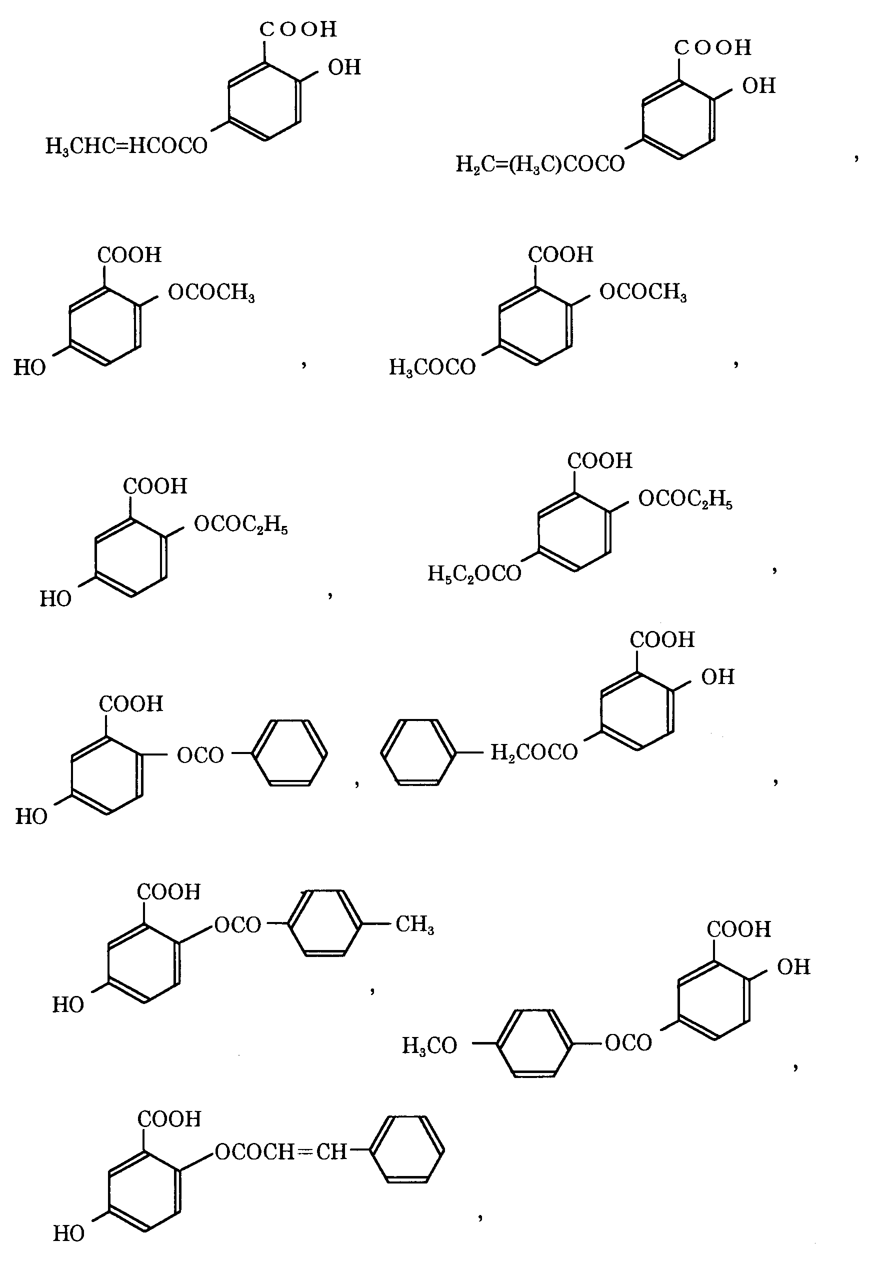

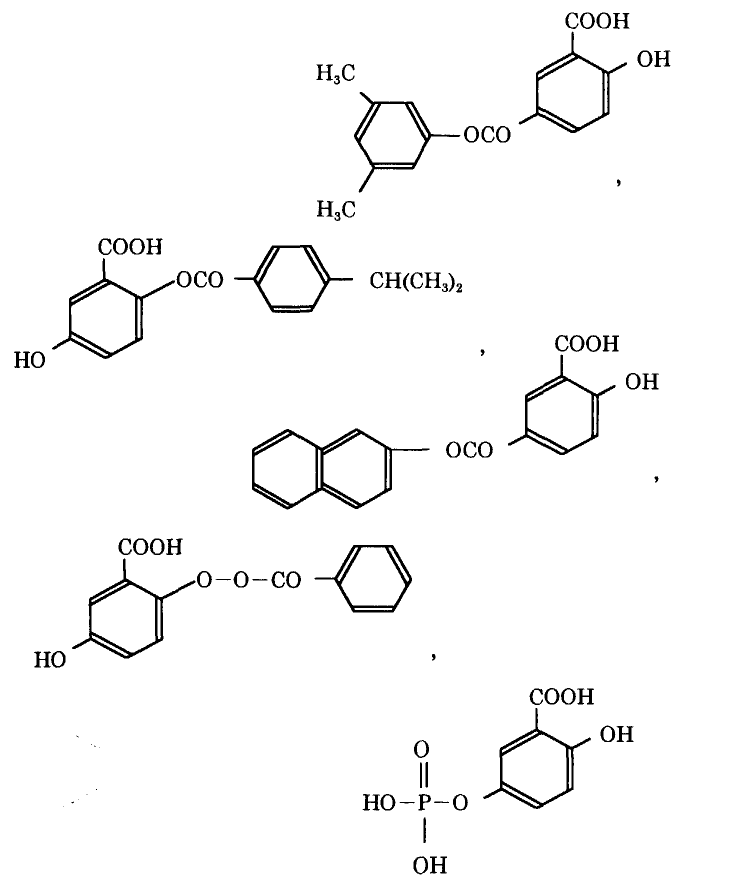

The compounds expressed by Formula (III): 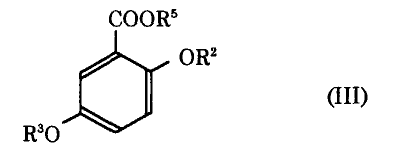 (Refer to the aforementioned description of R² and R³, although R² and R³ should not be hydrogen atoms in a same compound, and R⁵ is an alkyl group with 1 to 20 carbons or an allylalkyl group with seven to nine carbons.) Examples include the following:

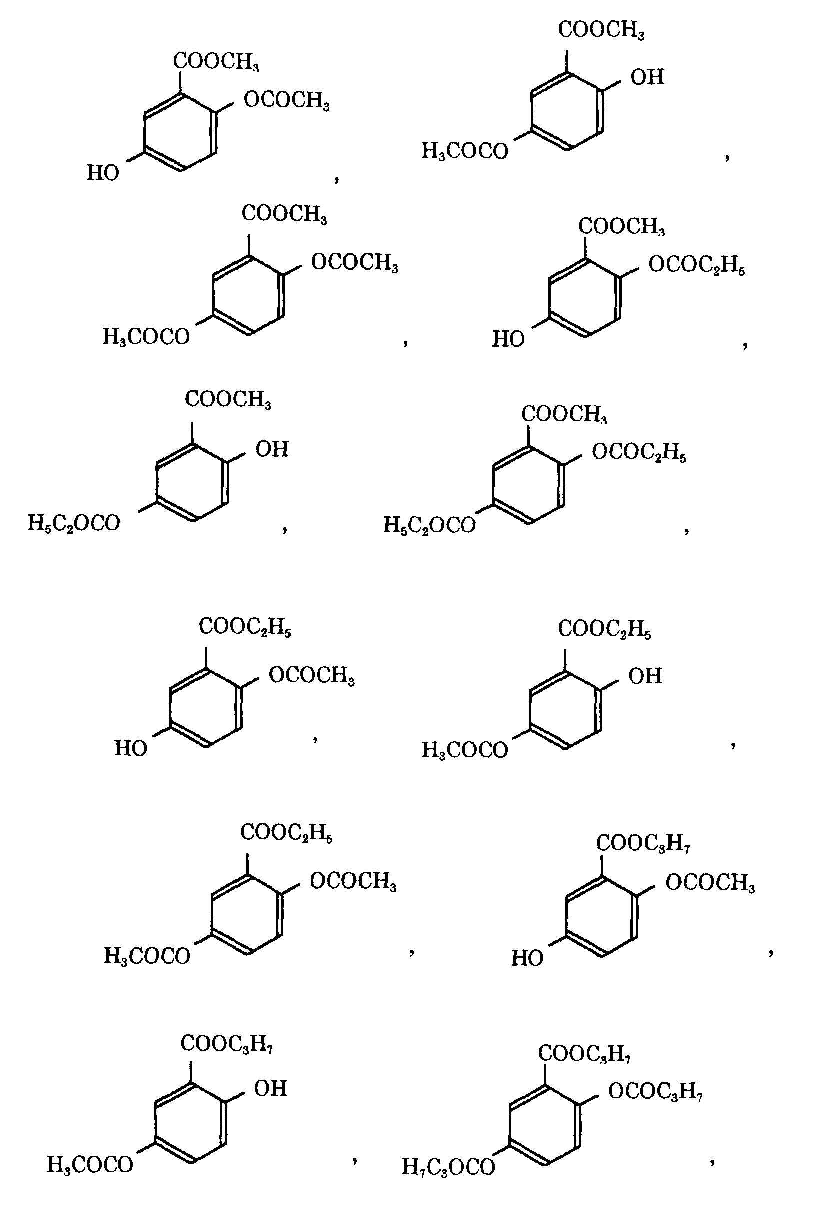

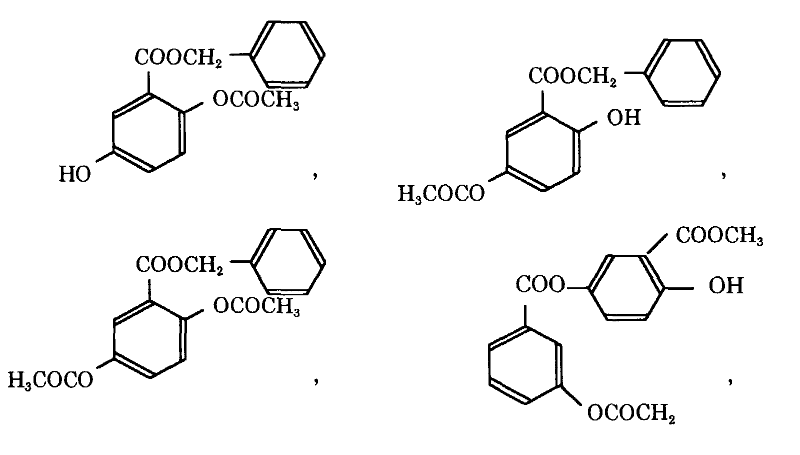

The compounds expressed by Formula (IV):

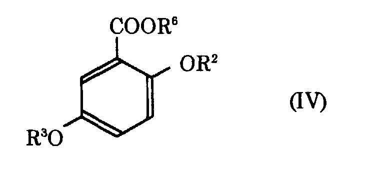

(Refer to the aforementioned description of R² and R³, although R² and R³ should not be hydrogen atoms in an identical compound, and R⁶ is an alkali metal atom or alkaline-earth metal.) Examples include the following:

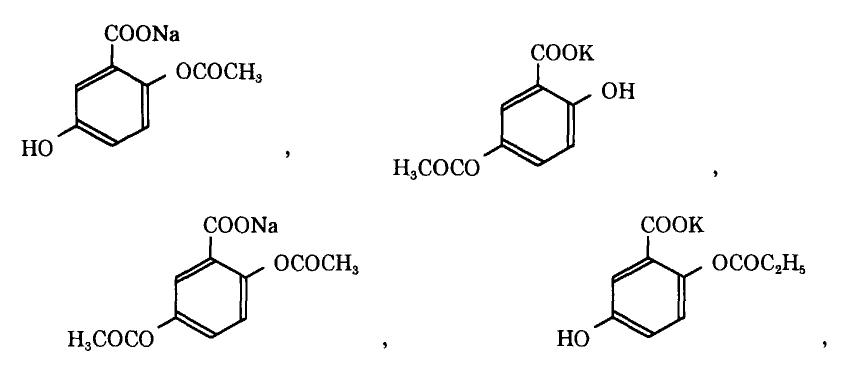

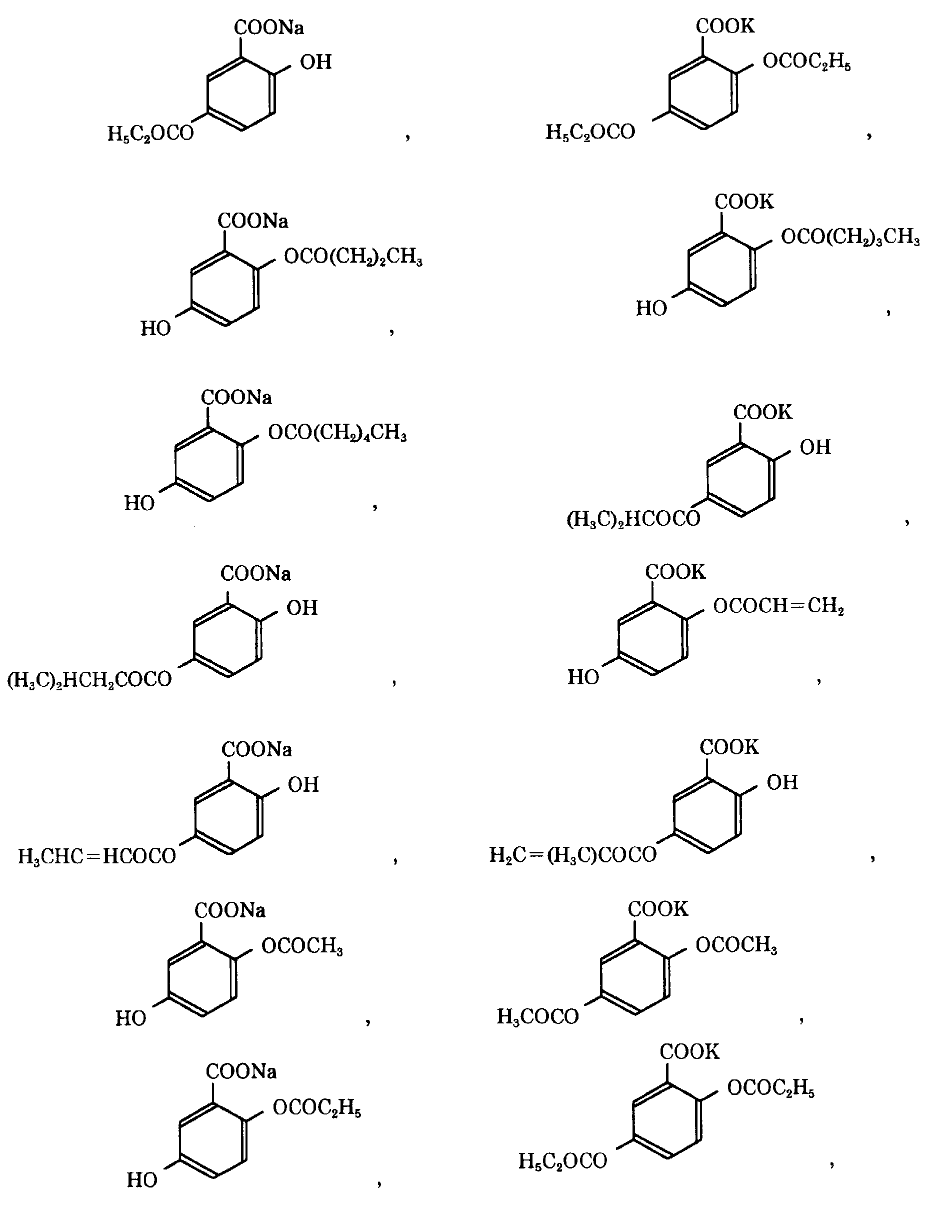

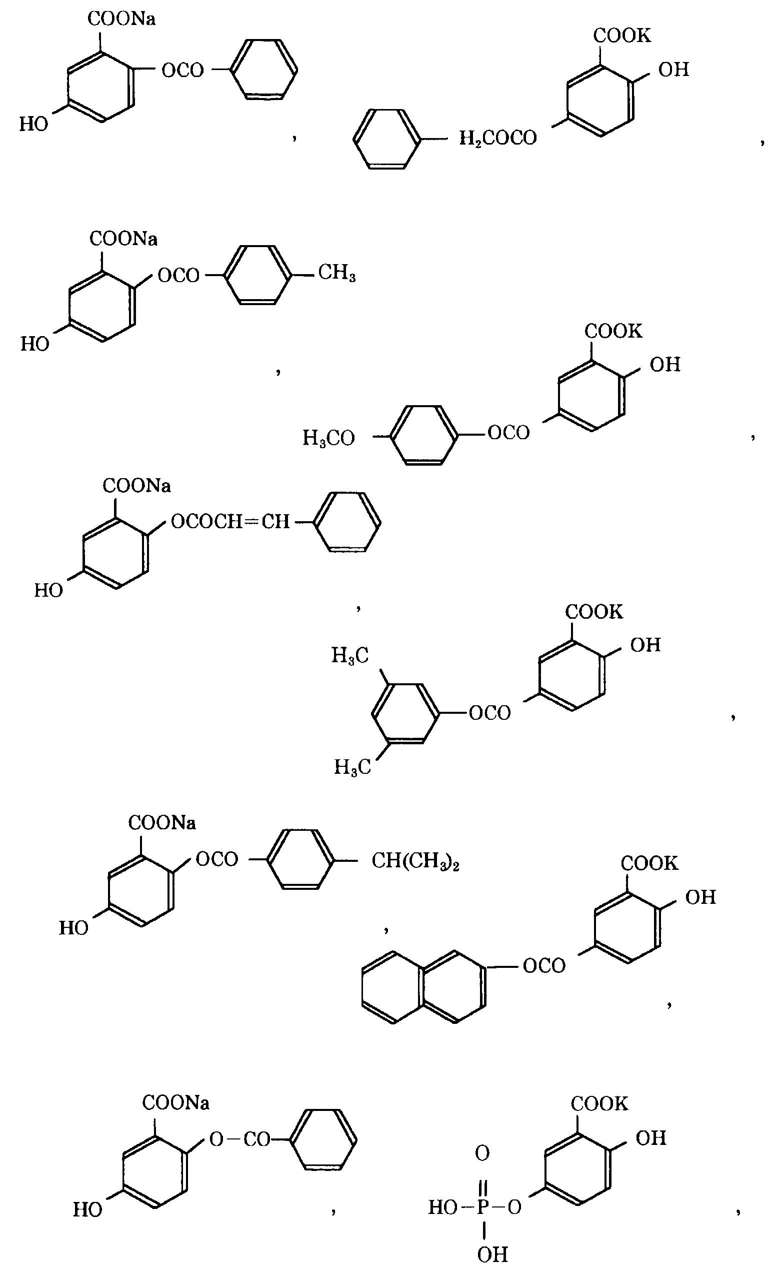

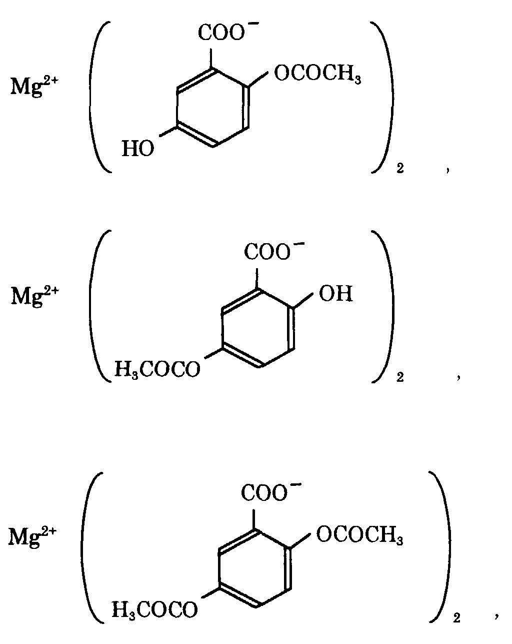

The compounds expressed by Formula (V): 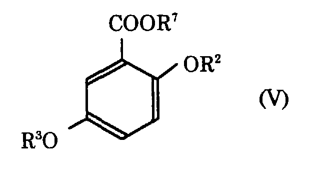 (Refer to the aforementioned description of R² and R³, although R² and R3 should not be hydrogen atoms in an identical compound) R7 is a group expressed by Formula (VI): 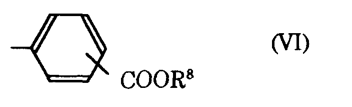 (In this formula, R8 is an alkyl group with one to three carbons.) Examples include the following:

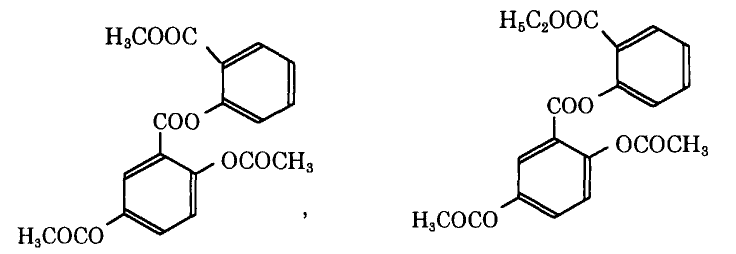

These compounds can be used either independently or in combinations of two or more. The following compounds are especially recommended because they are easily synthesized and easily obtained, can endow cosmetic products with better whitening, anti-inflammatory, and moisturizing effects, and are less irritant and more stable over long periods:

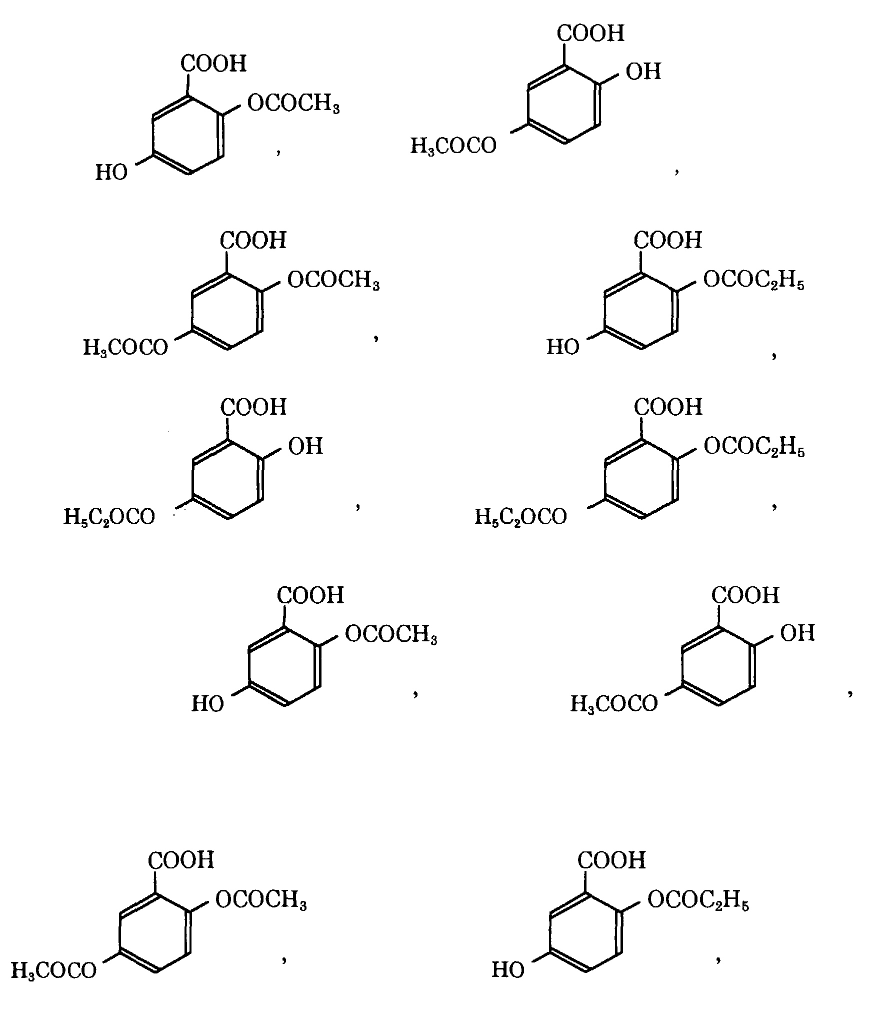

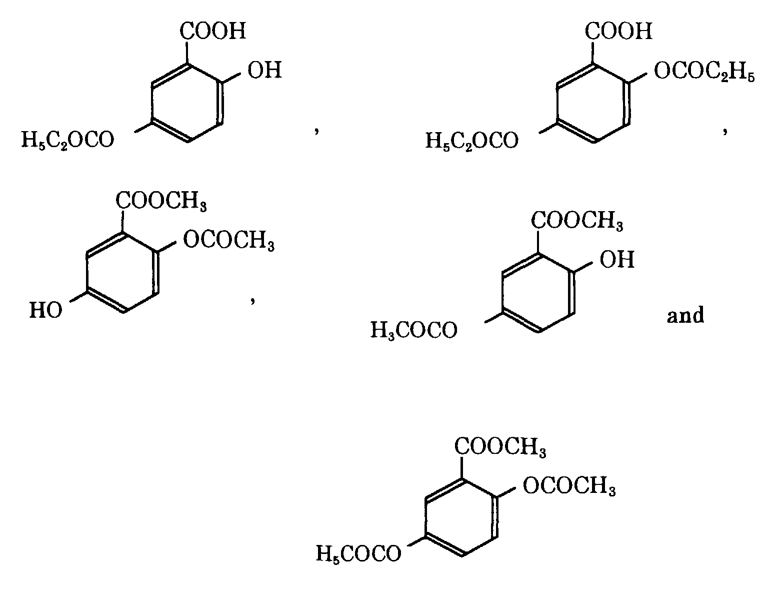

For the present invention, the 2,5-dihydroxy benzoic acid derivatives can be mixed directly to the cosmetic preparation. The amount of the 2,5-dihydroxy benzoic acid derivatives depends on the type of target preparation, and cannot therefore be specified. In order to obtain sufficient whitening, anti-inflammatory, and moisturizing effects, it is preferable to control the mixing ratio of the 2,5-dihydroxy benzoic acid derivatives in the preparation to at least 0.01 parts (per 100 parts of preparation in w/w; subsequent figures in this section are of the same ratio), or at least 0.05 parts for improved effects, or at least 0.1 parts for optimum effects. In order to prepare technically stable preparations, it is desirable to adjust the mixing ratio of the 2,5-dihydroxy benzoic acid derivatives in the preparation to at most 25 parts, or at most 10 parts for improved stability, or at most five parts for optimum stability.

The present invention already contains the 2,5-dihydroxy benzoic acid derivative(s). In addition to the 2,5-dihydroxy benzoic acid derivative(s) expressed by Formula (I), other agents such as a whitening agent commonly used in cosmetic preparations may be mixed to the present invention as appropriate.

Examples of these whitening agents are kojic acid, albutin, placenta extract, ascorbic acid, ascorbic acid phosphates such as L-ascorbyl magnesium phosphate, ascorbic acid derivatives, mulberry bark extract, glycyrrhiza extract, calcium pantetheine-S-sulphonate, etc., which can be mixed to the preparation either independently or in combinations of two or more.

To ensure sufficient whitening effect and stability in the preparation, it is recommended that the mixing ratio of the whitening agent(s) in the preparation be controlled to a range from 0.01 to 10 parts, especially to a range from 0.1 to 3 parts for the best effects. If the relevant whitening agents contain solid phase, the mixing ratio may be controlled to the aforementioned ratio by converting the amount of the solid phase. Adequate combination of such whitening agents and the 2,5-dihydroxy benzoic acid derivatives will result in a preparation with better whitening effect.

Examples of the aforementioned anti-inflammatory agents are glycyrrhizinic acid, glycyrrhizinic acid derivatives such as glycyrrhizinic acid dipotassium, allantoin and indomethacin, which can be mixed independently or in combinations of two or more.

For sufficient anti-inflammatory effect and stability in the preparation, it is recommended that the mixing ratio of the anti-inflammatory agent(s) in the preparation be controlled to a range from 0.01 to 10 parts, or to a range from 0.1 to 3 parts for the best effects. Combination of the anti-inflammatory agent(s) and the 2,5-dihydroxy benzoic acid derivatives will endow the preparation with better anti-inflammatory effect.

In addition to the aforementioned agents, a variety of other cosmetic ingredients such as excipients, aromatics, oils and fats, surfactants, moisturizing agents, pH regulators, thickeners, preservatives, antioxidants, ultra-violet absorbents, pigments, emulsifiers, plant and animal extracts, vitamins, amino acids, and the like may be mixed to the preparation as appropriate.

The oils and fats that are commonly used in cosmetic preparations are: plant oils such as liquid paraffin, paraffin, cetyl alcohol, avocado oil, olive oil, jojoba oil and palm oil; animal fats such as suet, lard, equine fat, turtle fat, mink fat, purslane oil and squalene and; synthetic oils such as methylpolysiloxane, behenyl alcohol, glyceryl tricaprylcaprinate, glyceryl torioctanate, glyceryl triisopalmitate and silicon oil.

The surfactants that are commonly used in the preparations include: anionic surfactants such as sodium lauryl sulfate, triethanolamine lauryl sulfate and diethanolamine lauryl sulfate; cationic surfactants such as stearyltrimethyl ammonium chloride, cetyltrimethyl ammonium chloride and benzalconium chloride; non-ionic surfactants such as glyceryl monostearate sorbitan monostearate, polyoxyethylene (20) sorbitan monostearate, polyoxyethylene hardened caster oil, sucrose esters, fatty acid amides.

The moisturizing agents that are commonly used in the preparations include: synthetic moisturizers such as glycerin, propylene glycol, 1,3-butylene glycol, sodium pyrrolidone carboxylate, pantetheine -S- sulfonate and; natural moisturizing agents such as hyaluronic acid, collagen, elastine, placenta extract, royal jelly, bacterial fermentation soup, chitin, chitosan, pectic substance and other extracts of plants and animals.

Organic acids and their salts such as citric acid and sodium citrate are used as the pH regulators.

The thickeners commonly used include carboxymethylcellulose, hydroxymethylcellulose, hydroxyethylcellulose, carboxyvinyl polymer, polyvinyl alcohol, tragacanth gum, sodium alginate and carrageenan.

The preservatives commonly used include p-hydroxybenzoates such as methyl p-hydroxybenzoate, ethyl p-hydroxybenzoate, propyl p-hydroxybenzoate and butyl p-hydroxybenzoate, phenoxyethanol, ethanol and dehydroxy acetic acid.

The antioxidants used include vitamin E, butyl hydroxytoluene (BHT), butyl hydoxyanisole (BHA), etc.

The pigments commonly used include red ion oxide, yellow ion oxide, black ion oxide, titanium oxide, nylon powder, serisite, mica and talc, etc.

The emulsifiers commonly used include soybean lecithin etc.

The excipients commonly used include mannnitol, sodium sulfate, etc.

The mixing ratio of the components can be modified as appropriate, as it depends on the purpose of the targeted preparation and cannot therefore be specified.

The description of the present invention, i.e. cosmetic preparation, is not fixed to any one option. Since the invention has a series of desirable characteristics such as preventing darkening, stains or freckles of the skin, inhibiting the occurrence of inflammation and maintaining the skin healthy and adequately moistened, the invention can be used for skin-care products such as creams, emulsions, lotions, essences, face-washes and facial-packs, make-up products such as lip-sticks, foundations, liquid foundations and pressed facial powder, and toiletry products such as shampoos, rinses, body soaps and soaps.

Since the 2,5-dihydroxy benzoic acid derivatives are absorbed through the skin and improve the skin condition with their moisturizing properties when the derivatives are dissolved in bath water, the invention can also be used for bathroom products such as bath agent. In order to obtain better moisturizing and skin conditioning effects of the 2,5-dihydroxy benzoic acid derivatives, the mixing ratio of the 2,5-dihydroxybenzoic acid derivatives in the preparation should be in a range from 0.01 to 10, or in a range from 0.1 to 1 parts for optimum effects. When such bath agents are actually in use, it is recommended that the concentration of the derivatives in the agent be controlled so that the amount of agent to be used is always 5-50 per 200L of bath water.

The 2,5-dihydroxybenzoic acid derivatives used in the invention are less irritant and more highly stable, while at the same time reducing tyrosinase activity in the pigment cell, inhibiting biosynthesis of tyrosinase enzyme protein and subsequently inhibiting melanin formation, preventing erythema caused by the reduction of cyclooxygenase activity preventing inflammation, and moisturizing the skin by controlling water retention. Therefore, the specific characteristics of the present invention, i.e. the cosmetic preparation, are the inhibition of occurrence of stains and freckles caused by increased melanin production, reduction of the occurrence of inflammation, improvement of the quality of the skin, whitening and moisturizing of the skin and contribution to the prevention of skin aging. Moreover, the invention also has the added property of being less irritant..

### Embodiments

A detailed description of the present invention, i.e. the cosmetic preparation, is given in the following section on the basis of the embodiments. However, the invention is not be limited to the following embodiments alone.
Synthesis 1: Synthesis of 2-(2'5'-diacetoxy-benzoiloxy) benzoic acid methyl ester 0.96g (4mM) of diacetylgentianic acid was dissolved in 10ml of chloroform. To this solution, 1.46ml (20mM) of thionyl chloride was mixed and the mixture was refluxed overnight under heating at 70° C. After cooling down, the solvent was evaporated under reduced pressure and diacetylgentianate chloride was obtained. Separately, 0.45ml (4mM) of methyl salicylate was dissolved in 10ml of chloroform, and 0.67ml (4.8nM) of triethylamine was mixed to this solution. The mixture was stirred for 30 minutes at ambient temperature. To the mixture, the synthesized chloride compound dissolved in 5ml of chloroform was added in the form of drops, and the mixture was stirred for two hours at room temperature for reaction. After distillation of the reaction solution under reduced pressure, the residue was applied to a silica-gel chromatography (mobile phase = chloroform / ether = 10/1) to obtain the target substance (recovery rate = 7.05%, melting point = 110-111° C).
Synthesis 2: Synthesis of 5-(2'-acetyloxy-benzoil) gentianic acid methyl ester 1.622g (9mM) of acetylsalicylic acid was dissolved in 15ml of chloroform. 3.28ml (45mM) of thionyl chloride was mixed to this solution and refluxed under heating at 70° C overnight. After cooling down, the solvent was evaporated under reduced pressure to obtain acetylsalicylate chloride. Separately 0.757g (4.5mM) of methyl gentianate was dissolved in 15ml of chloroform, to which 0.75ml (5.4mM) of triethylamine was then mixed. To this solution, the synthesized chloride compound, dissolved in 5ml of chloroform, was added in drops and stirred for two hours at room temperature for reaction. The reaction solution was distilled under reduced pressure and the residue obtained was applied to a silica gel chromatography (mobile phase = chloroform) to separate the target substance (recovery rate = 17.8%, melting point = 82-84° C).

### Experiments 1-4:

In accordance with the description below, Experiments 1, 3, and 4 were performed using 2,5-diacetoxy benzoic acid as expressed by the following formula, while Experiment 2 was performed using 2-(2',5'-diacetoxy-benzoil) benzoic acid methyl ester as described in Synthesis 1 for Experiment 2, both as the samples of the 2,5-dihydroxy benzoic acid derivatives.

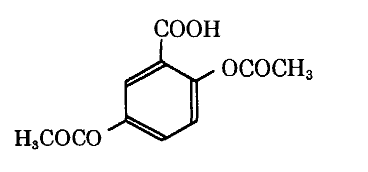

### Experiment 1: Inhibition of synthesis of tyrosinase enzymatic protein

Cultured B16 mouse melanoma cells (6x10⁴ cells) were inoculated to Eagle's minimum essential medium (MEM) containing 5% (v/v) of fetal bovine serum (FBS) and pre-incubated for one day under 5% CO₂ at 37° C before 4 days incubation with 0.1% D-glucosamine under the same conditions. Then the culture medium was replaced with one that contained 50mM of adenosine 3',5'-cyclic phosphate (c-AMP) and 5mM of 2,5-acetoxy benzoic acid as the sample, and incubated under 5% CO₂ for three days at 37° C.

For comparison, a separate series of incubation was conducted under the same conditions using the same culture medium without 2,5-diacetoxy benzoic acid, and served as a blank.

After the incubation, the cells were isolated from the medium by centrifugation and made into a pellet. The gross observation of pellets showed that the pellet obtained from the B16 mouse melanoma cells cultured with 2,5-diacetoxy benzoic acid was significantly whitened when compared with those obtained from the blank culture.

Then the culture samples were analyzed by SDS electrophoresis for detection of B16 mouse melanoma intracellular tyrosinase isozymes. The conditions of the electrophoresis were: gel = polyacrylamide gel (T=7.5% w/v, C=3% w/v); buffer solution = 0.025 M tris- 0.192 M glycine buffer solution (pH = 8.3) containing 0.1% (v/v) sodium dodecyl sulfate (SDS); amount of application = 20uL per well [the mixing ratio of the buffer A (38ml of 0.125M tris-HCl buffer, 10ml of glycerin, 2g of SDS and 3mg of bromphenol blue)/culture sample = 1/1]. The concentration of the sample was adjusted to 1600ug/ml.

The result of electrophoresis of the culture with 2,5-diacetoxy benzoic acid showed that, when compared with that of the blank culture, the band of T1-tyrosinase, an isozyme of tyrosinase in the B16 mouse melanoma cells, was significantly reduced, while the T3-tyrosinase band disappeared.

Therefore, because T1-tyrosinase itself significantly decreased before the transformation to T3-tyrosinase, the inhibition of biosynthesis of tyrosinase protein may be the main mechanism in the inhibitory effect on melanin production of 2,5-diacetoxy benzoic acid.

### Experiment 2: Inhibition of intracellular tyrosinase activity

Cultured B16 mouse melanoma cells were inoculated to a 96-well microplate (Corning Ltd.) at the rate of 8000 cells /well and pre-incubated in the Eagle's MEM containing 5% (v/v) FBS for one day under 5% CO₂ at 37° C. After the pre-incubation, the culture medium was replaced with the Eagle's MEM containing 5% FBS, also containing 2.5mM or 5mM 2,5-diacetoxy benzoic acid or 2.5mM of 2-(2',5'-diacetoxy-benzoiloxy) benzoic acid, whereupon incubation was continued for two days under 5% CO₂ at 37° C.

After the incubation, the medium was removed and a surfactant (Triton X-100) was mixed with the cells. Then 5mM of L-dopa or 0.2% of 3-(4,5-dimethylthiazol-2-yl)-2,5-diphenyltetrazorium bromide (MTT) was mixed for tyrosinase reaction at 37° C. Then the Dopa value and MTT test value were measured at the wavelength of 490nm and 570-630nm respectively using a microplate reader (Model 450, Bio Rad Ltd.).

For comparison, two series of culture with 2.5mM or 5mM of 2.5-dihydroxy benzoic acid instead of the compounds used in the present invention, as well as a culture without compound were prepared to serve as the positive controls and blank respectively.

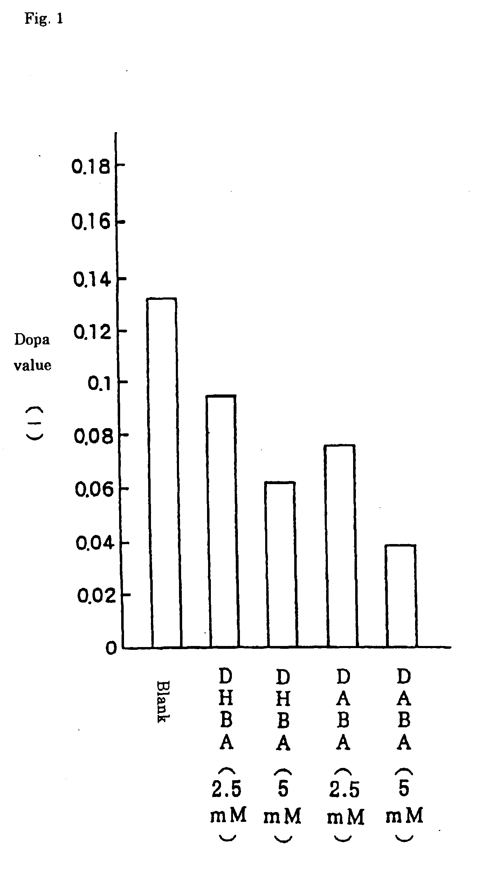

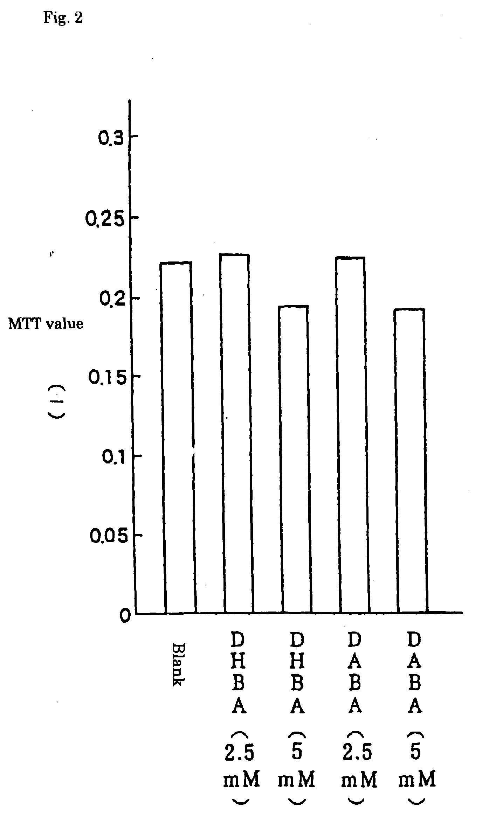

The Dopa values and MTT values are indicated in Fig. 1 and 2 respectively. In Fig. 1 and 2, DABA means 2,5-diacetoxy benzoic acid, DAPECSAM means 2-(2',5'-diacetoxy-benzoil) benzoate methyl ester, and DHBA means 2,5-dihydroxy benzoic acid.

It is believed that the higher the Dopa value, the higher the intracellular tyrosinase activity, while the lower the MTT test value, the lower the cultured B16 mouse melanoma cell activity.

The graph in Fig. 1 shows that, the Dopa value of the culture with 2,5-diacetoxy benzoic acid or with 2-(2',5'-diacetoxy-benzoiloxy) benzoic acid methyl ester was much lower than that of the blank or even than that of the culture with 2,5-dihydroxy benzoic acid at the same concentration. Therefore, it was indicated that the compounds in the present invention sufficiently inhibited the intracellular tyrosinase activity.

The graph in Fig. 2 indicates that, the MTT value of the culture with 2,5-diacetoxy benzoic acid or with 2-(2',5'-diacetoxy-benzoiloxy) benzoic acid methyl ester was not much lower than those of the cultures with 2,5-dihydroxy benzoic acid or of the blank. Therefore, it was evident that the compounds seldom inhibited the cultured B16 mouse melanoma cell activity.

It was concluded that 2,5-diacetoxy benzoic acid and 2-(2',5'-diacetoxy-benzoiloxy) benzoic acid methyl ester inhibited intracellular tyrosinase activity in cultured B16 mouse melanoma cells without inhibiting the cell activity to any significant extent.

For comparison, separate series of the experiment were carried out under the same conditions but using kojic acid and albutin, which are commonly used whitening agents. The results indicated that, within such a concentration range as did not inhibit the cell activity of the cultured B16 mouse melanoma, the inhibitory effect on intracellular tyrosinase of 2,5-diacetoxy benzoic acid used in the present invention was the same or even higher than that of these conventional whitening agents.

### Experiment 3: Inhibition of cyclooxygenase activity

A mixture solution was prepared using 3.57ml of 0.2M tris-HCl buffer solution containing 20mM of L-tryptophan, 0.03ml of 40mM hemoglobin solution, 0.20ml of 400unit/ml cyclooxygenase solution, and 1.00ml of 5mM or 10mM 2.5-diacetoxy benzoic acid solution.

For comparison, a mixture using 1.00ml of purified water instead of 1.00ml of 2,5-diacetoxy benzoic acid was prepared to serve as a blank.

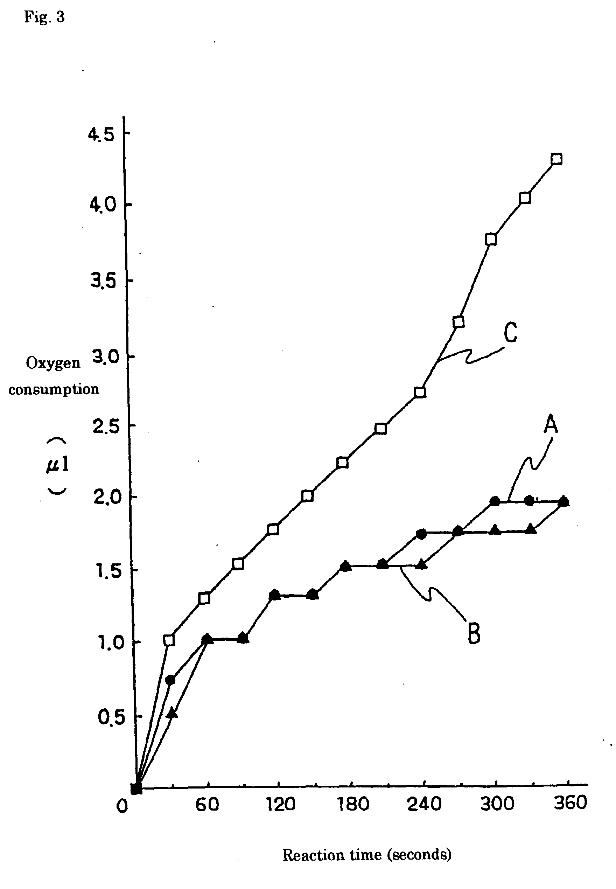

The mixture was incubated for five minutes at 37° C, and then 0.20ml of 0.2M tris-HCl buffer solution containing 2mM of arachidonic acid was added to the mixture for cyclooxygenase reaction. Immediately after the reaction started, the amount of dissolved oxygen in the reaction solution was measured using a biological oxygen monitor (YSI Model 5300, Meridian Instruments Far East, Ltd.). On the basis of the measurement of the dissolved oxygen level, the change in oxygen consumption attendant on the progress of the reaction time was determined. The results are shown in Fig. 3.

In Fig. 3, Graphs A and B show the results from the measurement of the reaction solutions of 2,5-diacetoxy benzoic acid with 5mM and 10mM respectively while Graph C shows the result from the blank using purified water.

It is believed that the less the oxygen consumption, the more the cyclooxygenase was inhibited.

It is also known from the graphs in Fig. 3 that, when compared with the blank, the increase in oxygen consumption attendant upon progress of reaction time was much lower in the reaction with 2,5-diacetoxy benzoic acid, and hence 2,5-diacetoxy benzoic acid has a significant inhibitory effect on cyclooxygenase activity.

### Experiment 4: Irritant properties

Cultured rabbit cornea cells (SIRC-C) were inoculated to a 96-well microplate (Corning Ltd.) at the rate of 5000 cells/well, and pre-incubated with Eagle's MEM containing 5% (v/v) FBS for one day under 5% CO₂ at 37° C. Then 0.2ml of Eagle's MEM containing 0.05% neutral red was added to the culture medium and incubation was continued for two days under the same conditions. Furthermore, 2,5-diacetoxy benzoic acid was added to the culture medium at the concentrations of 0.025% (w/v) (10.5mM), 0.05% (w/v) (21mM), 0.1% (w/v) (42mM) or 0.2% (w/v) (84mM) and incubated again for two days under 5% CO 2 at 37° C.

For comparison, cultures with 30ug/ml, 60ug/ml or 90ug/ml of sodium dodecyl sulfate instead of 2,5-diacetoxy benzoic acid and without any compound were prepared and served as the positive control and the blank respectively.

The cultures were tested by analyzing SIRC-C neutral red intake, which is one of the alternative methods of eye-irritation test, and the midpoint cytotoxicity (NR₅₀) value was derived from the neutral red intake rate to evaluate the potential irritation effect. The neutral red intake rate of each culture is shown in Table 1.

In Table 1, DABA means 2,5-diacetoxy benzoic acid and SDS means sodium dodecyl sulfate.

It is believed that, since the neutral red intake decreases with the increase in the irritation of the compound to SIRC-C, the higher the NR₅₀ concentration of the sample solution, the lower the irritation to the cells.

**Table 1**

| Culture | | Intake rate of neutral red (-) |
|---|---|---|
| Concentration of DABA (w/v%) | 0 | 0.83 |
| | 0.025 | 0.993 |
| | 0.05 | 0.914 |
| | 0.1 | 0.708 |
| | 0.2 | 0.145 |
| Concentration of SDS (µg/ml) | 0 | 0.83 |
| | 30 | 0.943 |
| | 60 | 0.389 |
| | 90 | 0.007 |

Table 1 indicates that the intake rate of neutral red was 0.943 and 0.389 when the concentration of sodium dodecyl sulfate was 30ug/ml and 60ug/ml respectively. Therefore, it was estimated that the NR₅₀ value in SIRC-C of sodium dodecyl sulfate was about 60ug/ml. Under the same conditions, the intake rate was 0.993 and 0.708-0.145 when the concentration of 2,5-diacetoxy benzoic acid was 0.025% (w/v) and 0.1-0.2% (w/v) respectively. Hence, it was assumed that the NR₅₀ value of 2,5-diacetoxy benzoic acid was 1000-2000ug/ml. It was shown that 2,5-diacetoxy benzoic acid was much less irritating to the cells than sodium dodecyl sulfate.

### Experiment 5:

The compound expressed by Formula A was used as an example of the 2,5-dihydroxy benzoic acid derivatives to evaluate its inhibitory effect on intracellular tyrosinase activity in accordance with the same protocol as followed in Experiment 2.

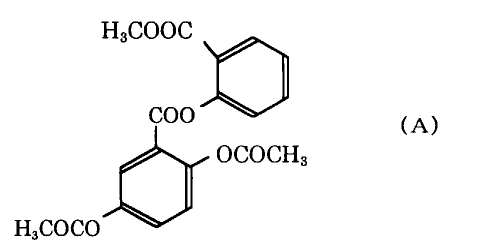

However, the concentration was set to 2.5mM and the compound was dissolved in dimethyl sulfoxide before being applied to the culture medium.

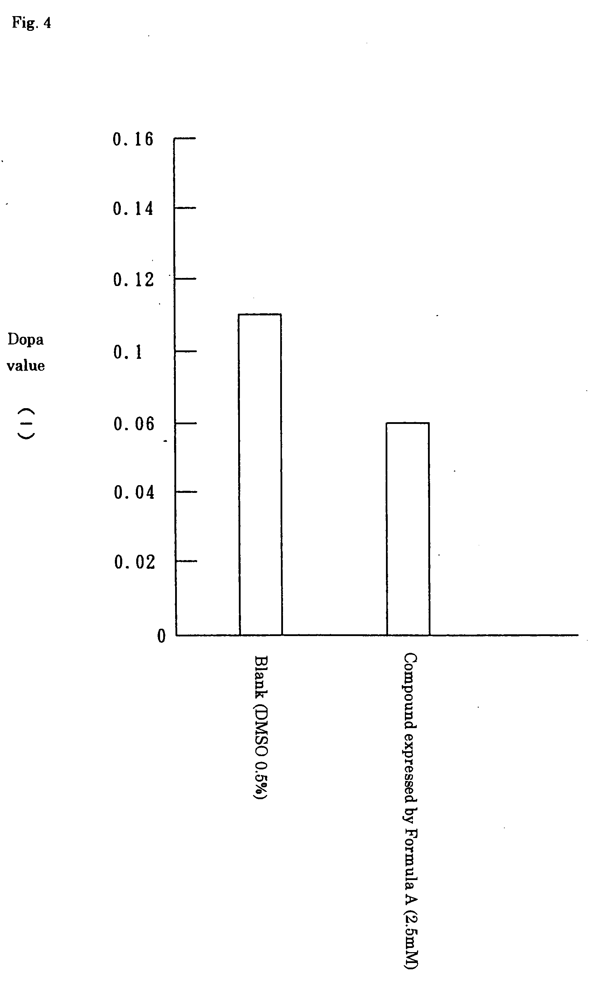

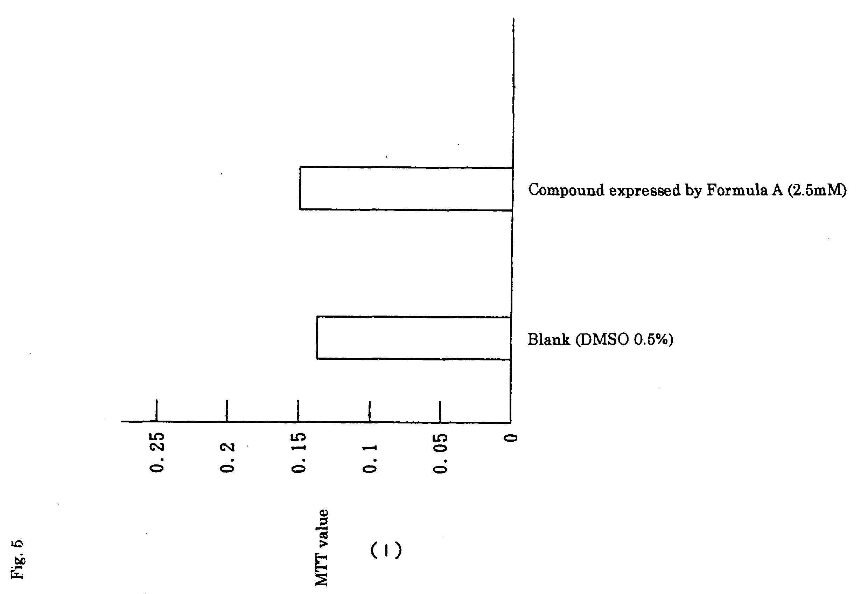

The medium containing dimethyl sulfoxide was used for the blank culture. The results are shown in the graphs in Fig. 4 (Dopa levels) and Fig. 5 (MTT levels).

The graphs show that the compound significantly inhibited intracellular tyrosinase activity but not the cell activity.

### Formulations 1 to 23 and Control Formulation 1

Described in this section are a variety of formulations containing 2,5-dihydroxy benzoic acid derivatives expressed by the following formula, in addition to a control formulation for comparison with Formulations 1 to9.

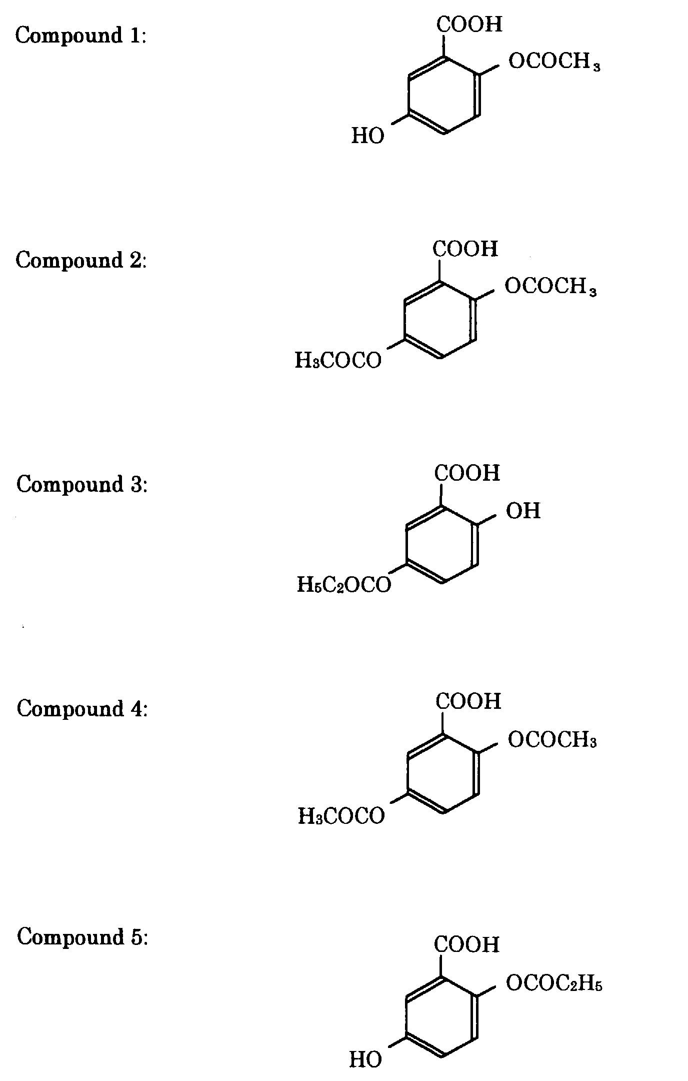

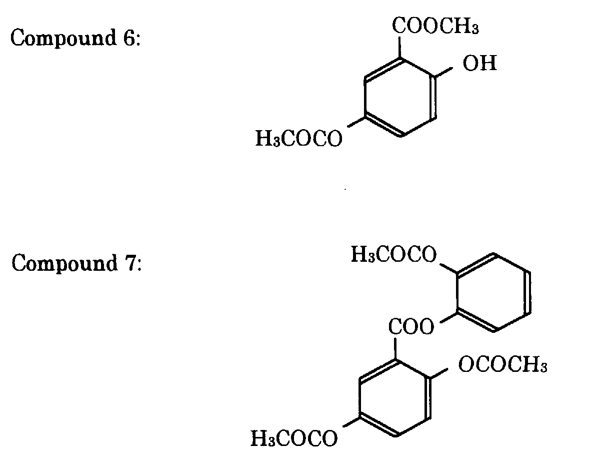

### Formulation 1: Cream

| 〈Components A〉 | Mixing ratio (parts) |
|---|---|
| Liquid paraffin | 5.0 |
| Hexalan (trade name of glyceryl trioctate, Technoble Co., Ltd.) | 4.0 |
| Paraffin | 5.0 |
| Cetyl alcohol | 2.0 |
| Glyceryl monostearate | 2.0 |
| Polyoxyethylene (20) | 6.0 |
| sorbitan monostearate | |
| Butyl paraben | 0.1 |

| 〈Components B〉 | |
|---|---|
| Compound 1 | 5.0 |
| Glycerin | 5.0 |
| Sodium carboxymethyl cellulose | 0.1 |
| Methyl paraben | 0.1 |
| Moisten C (trade name of NMF component, Technoble Co., Ltd.) | 1.0 |
| Purified water | Amount needed to bring the total to 100.0 parts |

| 〈Component C〉 | |
|---|---|
| Aromatics | As appropriate |

Components A and B were separately heated to at least 80° C before they were mixed together and stirred. The mixture was cooled to 50° C and Component C was mixed to the mixture and stirred thoroughly to prepare a uniform cream.

### Formulation 2: Cream

A uniform cream formulation was prepared following the prescription of Formulation 1 but using Compound 2 (1.0 parts) instead of Compound 1 (5.0 parts).

### Formulation 3: Cream

A uniform cream formulation was prepared following the prescription of Formulation 1 but using Compound 2 (0.5 parts) and Compound 3 (0.5 parts) instead of Compound 1 (5.0 parts).

### Formulation 4: Cream

A uniform cream formulation was prepared following the prescription of Formulation 1 but using Compound 4 (1.0 parts) and Compound 5 (1.0 parts) instead of Compound 1 (5.0 parts).

### Formulation 5: Cream

A uniform cream formulation was prepared following the prescription of Formulation 1 but using Compound 6 (0.1 parts) instead of Compound 1 (5.0 parts).

### Formulation 6: Cream

A uniform cream formulation was prepared following the prescription of Formulation 2 but using L- ascorbyl magnesium phosphate (1.0 parts) in addition to Component B.

### Formulation 7: Cream

A uniform cream formulation was prepared following the prescription of Formulation 2 but using potassium glycyrrhizate (1.0 parts) in addition to Component B.

### Formulation 8: Cream

A uniform cream formulation was prepared following the prescription of Formulation 2 but using kojic acid (0.5 parts) and L- ascorbyl magnesium phosphate (0.5 parts) in addition to Component B.

### Formulation 9: Cream

A uniform cream formulation was prepared following the prescription of Formulation 1 but using Compound 7 (0.1 parts) instead of Compound 1 (5.0 parts).

### Control Formulation 1: Cream

A cream formulation was prepared following the prescription of Formulation 1 but using purified water instead of Compound 1.

### Formulation 10: Emulsion

| 〈Components A〉 | Mixing ratio (parts) |
|---|---|
| Liquid paraffin | 6.00 |
| Hexalan (trade name of glyceryl trioctate, Technoble Co., Ltd.) | 4.00 |
| Jojoba oil | 1.00 |
| Polyoxyethylene (20) sorbitan monostearate | 2.00 |
| Soybean lecithin | 1.50 |
| Methylparaben | 0.15 |
| Ethylparaben | 0.03 |

| 〈Components B〉 | |
|---|---|
| Compound 2 | 2.00 |
| Glycerin | 3.00 |
| 1,3-butylene glycol | 2.00 |
| Sodium carboxymethyl cellulose | 0.30 |
| Sodium hyaluronate | 0.01 |
| Purified water | amount which makes the total 100.00 parts |

| 〈Component C〉 | |
|---|---|
| Aromatics | Trace |

Components A and B were separately heated up to at least 80° C before mixed together and stirred. After the mixture was cooled to 50° C, Component C was mixed and stirred further until the final mixture became a uniform emulsion.

### Formulation 11: Emulsion

A uniform emulsion was prepared following the prescription of Formulation 10 but using Compound 2 (0.50 parts) and Compound 5 (0.20 parts) instead of using 2.00 parts of Compound 2.

### Formulation 12: Emulsion

A uniform emulsion was prepared following the prescription of Formulation 10 but using Compound 7 (0.50 parts) instead of Compound 2 (2.00 parts).

### Formulation 13: Lotion

| 〈Components〉 | Mixing ratio (parts) |
|---|---|
| Ethanol | 10.0 |
| Glycerin | 3.0 |
| 1,3-butylene glycol | 2.0 |
| Methylparaben | 0.2 |
| Citric acid | 0.1 |
| Sodium citrate | 0.3 |
| Carboxyvinyl polymer | 0.1 |
| Compound 2 | 1.0 |
| Aromatics | Trace |
| Purified water | Amount needed to bring the total to 100.00 parts |

All the components were mixed to prepare a uniform lotion formulation.

### Formulation 14: Lotion

A uniform lotion formulation was prepared following the prescription of Formulation 13 but using L-ascolbyl magnesium phosphate (0.5 parts) in addition to the components listed above.

### Formulation 15: Facial pack

| 〈Components〉 | Mixing ratio (parts) |
|---|---|
| Polyvinyl alcohol | 15.0 |
| Hydroxymethyl cellulose | 5.0 |
| Propylene glycol | 5.0 |
| Ethanol | 10.0 |
| Methylparaben | 0.1 |
| Compound 2 | 0.5 |
| Aromatics | As appropriate |
| Purified water | Amount needed to bring the total to 100.00 parts |

All the components were mixed to prepare a uniform facial pack formulation.

### Formulation 16: Facial pack

A uniform facial pack formulation was prepared following the prescription of formulation 15 but using indomethacin (0.5 parts) in addition to the components listed above.

### Formulation 17: Face wash

| 〈Components〉 | Mixing ratio (parts) |
|---|---|
| Stearic acid | 15.0 |
| Lauric acid | 5.0 |
| Myristic acid | 15.0 |
| Glyceryl monostearate | 4.0 |
| Potassium hydroxyside | 7.0 |
| Glycerin | 8.0 |
| Compound 1 | 0.2 |
| Compound 2 | 1.0 |
| Methylparaben | 0.2 |
| Purified water | Amount needed to bring the total to 100.00 parts |

The components were mixed at 85° C to prepare a uniform face-wash formulation.

### Formulation 18: Lip stick

| 〈Components A〉 | Mixing ratio (parts) |
|---|---|
| Caster oil | 50.0 |
| Octyldodecanol | 5.0 |
| Lanolin | 5.0 |
| Liquid lanolin | 5.0 |
| Bees wax | 4.0 |
| Ozokerite | 7.0 |
| Candellila wax | 2.0 |
| Carnauba wax | 1.0 |

| 〈Components B〉 | |
|---|---|
| Compound 2 | 0.3 |
| Titanium oxide | 1.0 |
| Coloring matters (such as Red pigment No. 201) | 4.0 in total |

| 〈Component C〉 | |
|---|---|
| Aromatics | Trace |

Components A and B were separately warmed before mixing, and then the mixture was stirred. The mixture was warmed again before addition of Component C. Then the final mixture was poured into a mold and cooled to prepare a lipstick formulation.

### Formulation 19: Liquid foundation

| 〈Components A〉 | Mixing ratio (parts) |
|---|---|
| Stearic acid | 2.4 |
| Propylene glycol monostearate | 2.0 |
| Cetostearyl alcohol | 0.2 |
| Liquid lanolin | 2.0 |
| Liquid paraffin | 3.0 |
| Isopropyl myristate | 8.5 |
| Propylparaben | As appropriate |

| 〈Components B〉 | |
|---|---|
| Sodium carboxymethyl cellulose | 0.2 |
| Bentonite | 0.5 |
| Propylene glycol | 4.0 |
| Triethanolamine | 1.1 |
| Methylparaben | As appropriate |
| Compound 2 | 1.0 |
| Purified water | Amount needed to bring the total to 100.00 parts |

| 〈Components C〉 | |
|---|---|
| Titanium oxide | 8.0 |
| Talc | 4.0 |
| Coloring pigment | As appropriate |

Components A and B were warmed separately, and mixed and stirred. Then the mixture was warmed and Component C was mixed. The final mixture was poured into a mold, mixed and stirred until it cooled to room temperature to prepare a uniform liquid foundation formulation.

### Formulation 20: Liquid foundation

A uniform liquid foundation formulation was prepared following the prescription of Formulation 19 but using Compound 5 instead of Compound 2, and also using albutin (0.5 parts) and kojic acid (0.5 parts) in addition to Components B.

### Formulation 21: Bath agent

| 〈Components〉 | Mixing ratio (parts) |
|---|---|
| Sodium sulfate | 37.0 |
| Sodium hydrogen carbonate | 55.0 |
| Borax | 2.0 |
| Sodium carboxymethyl cellulose | 1.0 |
| Coloring matter 201 | Trace |
| Aromatics | Trace |
| Compound 2 | 5.0 |

The components were mixed to prepare a uniform bath agent formulation.

### Formulation 22: Soap

| 〈Components〉 | Mixing ratio (parts) |
|---|---|
| Beef fat/palm oil soap (80/20, w/w) | 66.0 |
| Sodium dilauryl phosphate | 2.0 |
| Sodium monolauryl phosphate | 17.0 |
| n-cocoaminopropyl betaine | 10.0 |
| Compound 2 | 2.0 |
| Compound 4 | 3.0 |

The components were mixed to prepare a uniform soap formulation.

### Formulation 23: Soap

A uniform soap formulation was prepared following the prescription of Formulation 22 but changing the mixing ratio of Compound 4 to 1.5 parts from 3.0 parts and using allantoin (1.5 parts) in addition to the components.

The following tests were conducted using the preparations obtained from the prescriptions described above.

### Test 1

The test was carried out to compare the inhibitory effects of daily use of the creams obtained from Formulations 1, 2, 3, 7, and 9 and Control formulation 1 on the chromatosis (pigmentation) in the skin.

Some 20 healthy adults (men and women) between the ages of 20 to 60 years old were randomly selected for the test. They were asked to apply a portion of 0.05g of each cream on the skin in their right and left armpits once daily for one month. Gross observation was made on the pigmentation in the armpit of the subjects. Evaluation was performed according to the following criteria and the result is given in Table 2:

### Criteria

(When compared with the appearance of the skin before the application of the creams)
A: Pigmentation disappeared.
B: Pigmentation was significantly reduced.
C: Pigmentation somewhat decreased.
D: No significant change.
E: Pigmentation increased.

**Table 2**

| Formulation No. | Evaluation of pigmentation (number of subjects) | | | | |
|---|---|---|---|---|---|
| | A | B | C | D | E |
| 1 | 7 | 9 | 4 | 0 | 0 |
| 2 | 7 | 8 | 4 | 1 | 0 |
| 3 | 5 | 9 | 5 | 1 | 0 |
| 7 | 8 | 10 | 2 | 0 | 0 |
| 9 | 6 | 9 | 4 | 1 | 0 |
| Control formulation 1 | 0 | 0 | 5 | 13 | 2 |

Table 2 indicates that the cream of Control formulation 1 without the 2,5-dihydroxy benzoic acid derivatives used in the present invention did not inhibit pigmentation, but the creams from formulations 1, 2, 3 and 7 removed or reduced pigmentation. Therefore, it was concluded that the creams from Formulations 1, 2, 3 and 7 had sufficient inhibitory effect on pigmentation.

In Test 1, there was no subject who developed abnormal change on the region where the creams from Formulations 1, 2, 3, 7 and 9 were applied.

### Test 2

The test was carried out to compare the inhibitory effects of the creams from Formulations 1,2,3,7 and 9 and Control formulation 1 on the onset of erythema and pigmentation induced by ultra-violet radiation.

Some 20 healthy adults (male and female) between the ages of 20 to 60 years old were randomly selected for the test. Two 1 × 1cm2 sites for UV radiation were marked on the medial side of the forearm of the subjects. The minimum erytematous dose (MED) of UV light was determined for each subject in advance, and the amount equivalent to the MED was radiated to each subject using an UV-B lamp (FL20-SE, Toshiba Co., Ltd.) once a day (in the morning) for three consecutive days.

Starting from the first day of UV radiation, a portion of about 0.025g of the creams from Formulations 1,2,3,7 and 9 and Control formulation 1 was applied to the radiated sites twice a day, immediately after the radiation and in the evening during the period of radiation (i.e. the first three days), and then in the morning and in the evening after the termination of radiation (i.e. from the fourth day to the end of the test), everyday for one month.

After one month of application, gross observation was made for occurrence of erythema and pigmentation of the radiated sites of the individual subjects. The inhibitory effect on onset of erythema and pigmentation was evaluated according to the following criteria and the result is given in Table 3:

### Criteria

(When compared with the appearance of the skin before application of the creams:)
A: Erythema and pigmentation were not observed.
B: Erythema and pigmentation significantly decreased.
C: Erythema and pigmentation somewhat decreased.
D: No significant change.
E: Erythema and pigmentation increased.

**Table 3**

| Formulation No. | Evaluation of onset of erythema and pigmentation (number of subjects) | | | | |
|---|---|---|---|---|---|
| | A | B | C | D | E |
| 1 | 7 | 10 | 3 | 0 | 0 |
| 2 | 6 | 10 | 4 | 0 | 0 |
| 3 | 5 | 9 | 5 | 1 | 0 |
| 7 | 8 | 9 | 3 | 0 | 0 |
| 9 | 6 | 9 | 5 | 0 | 0 |
| Control formulation 1 | 0 | 0 | 5 | 14 | 1 |

Table 3 indicates that the cream of Control formulation 1 without the 2,5-dihydroxy benzoic acid derivatives used in the present invention, did not inhibit onset of erythema and pigmentation, but the creams from formulations 1, 2, 3, 7 and 9 removed or reduced erythema and pigmentation. Therefore, it was concluded that the creams from Formulations 1, 2, 3, 7 and 9 had sufficient inhibitory effect on the onset of erythema and pigmentation.

In Test 2, there was no subject who developed abnormal change on the region where the creams from Formulations 1,2,3,7 and 9 were applied.

### Test 3

The test was carried out to compare the moisturizing effects of the creams from Formulations 1,2,3,7 and 9 and control formulation 1.
Some 20 healthy adults (male and female) between the ages of 20 to 60 years old were randomly selected for the test. The subjects were asked to apply a portion of 0.05g of each cream on the skin in the medial side of their right and left forearms twice a day, every day for one month. Then the water content of the skin in the medial side of their both forearms was measured using an impedance meter (SKICON 200, IBS Ltd.). Evaluation was performed according to the following criteria and the result is given in Table 4:

### Criteria

(When compared with the water content before application of the creams:)
A: Water content increased by 50% or more (in weight) compared with the time before the creams were applied.
B: Increase in water content was 20% or more and less than 50% (in weight).
C: Increase in water content was 10% or more and less than 20% (in weight).
D: Increase in water content was less than 10% (in weight) or change in water content was absent.
E: Water content decreased.

**Table 4**

| Formulation No. | Evaluation of water content (number of subjects) | | | | |
|---|---|---|---|---|---|
| | A | B | C | D | E |
| 1 | 7 | 9 | 4 | 0 | 0 |
| 2 | 5 | 10 | 4 | 1 | 0 |
| 3 | 5 | 9 | 5 | 1 | 0 |
| 7 | 7 | 10 | 3 | 0 | 0 |
| 9 | 5 | 9 | 5 | 1 | 0 |
| Control formulation 1 | 0 | 0 | 5 | 13 | 2 |

Table 4 indicates that the cream of Control formulation 1 without the 2,5-dihydroxy benzoic acid derivatives used in the present invention evidenced little moisturizing effect, whereas the creams from formulations 1, 2, 3, 7 and 9 increased the skin water content by 10 to 50% or more (in weight). Therefore, it was concluded that the creams of Formulations 1, 2, 3, 7 and 9 restored the moisture of the akin and had sufficient moisturizing effect.

In Test 3, there was no subject who developed abnormal change on the region where the creams from Formulations 1, 2, 3, 7 and 9 were applied.

There was no change in the appearance of the creams of Formulations 1, 2, 3, 7 and 9 within one month after preparation.

### Advantages of the present invention

2,5-dihydroxy benzoic acid derivatives, which are expressed by Formula (1), have little inhibitory effect on the cell activity, reduce biosynthesis of melanin by reducing pigment cell intracellular tyrosinase activity (an index of potential whitening effect) and by inhibiting production of tyrosinase enzyme protein, while also preventing erythema and pigmentation induced by UV-light radiation. In addition to these effects, the derivatives reduce cyclooxygenase activity (an index of potential anti-inflammatory effect) and have some advantageous effects such as increasing skin water content and subsequently increasing moisturizing effect.

Therefore, the present invention, a cosmetic preparation, which contains the 2,5-dihydroxy benzoic acid derivatives prevents the occurrence of stains and freckles, improves the skin conditions with its anti-inflammatory effect, improves the quality of the skin by imparting moisture, maintains the skin healthy and clean, and thus contributes to the prevention of skin aging.

The present invention, i.e. the cosmetic preparation, has a variety of desirable properties including whitening, anti-inflammatory, and moisturizing effects, as well as the attendant property of preventing skin aging. In addition, the invention is low irritant, stable on the skin, and also stable when stored.

## Claims

1. A cosmetic preparation that contains the 2,5-dihydroxy benzoic acid derivatives which are expressed by Formula (I): 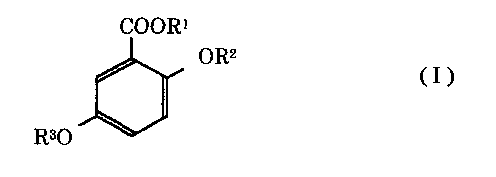 [In formula (1), R¹ is a hydrogen atom, an alkyl group with one to 20 carbons, an allylalkyl group with seven to nine carbons, an alkali metal atom or an alkaline earth metal atom; R² and R³ is independently a hydrogen atom, a group expressed by the formula -COR⁴ (R⁴ is an alkyl group with one to five carbons, an alkenyl group with one to three carbons, a methoxy group or an ethoxy group; R⁶ is a hydrogen atom or an alkyl group with one to three carbons) or a phosphoryl group. However, R² and R³ should not be hydrogen atoms in an identical compound.]

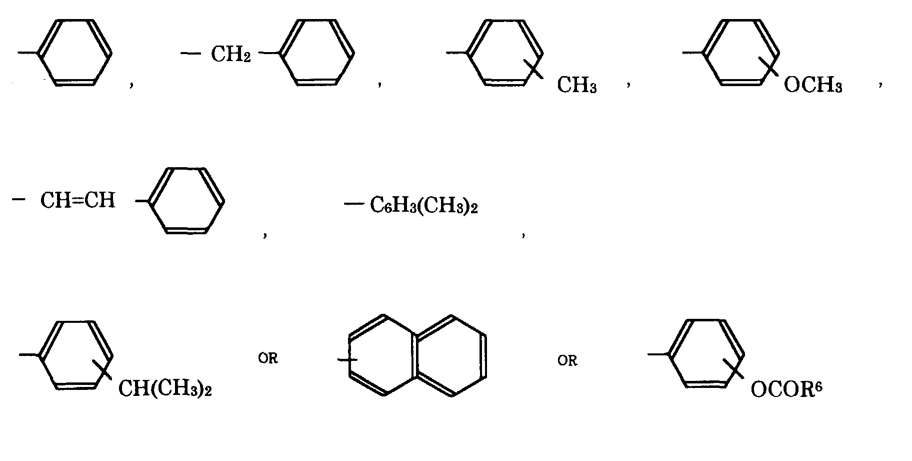

2. A cosmetic preparationdescribed in Section 1, which contains the 2,5-dihydroxy benzoic acid derivatives 2-acetoxy-5-hydroxy benzoic acid, 2,5-diacetoxy benzoic acid, 2-hydroxy-5-propionyloxy benzoic acid, 2,5-diacetoxy benzoic acid, 2-propionyloxy-5-hydroxy benzoic acid and 2-hydroxy-5-acetoxy benzoic acid methyl esters and/or 2-(2',5'-diacetoxybenzoiloxy) benzoic acid methyl ester.
